(19) Europäisches Patentamt
European Patent Office
Office européen des brevets

(11) **EP 3 832 303 A1**

(12) **EUROPEAN PATENT APPLICATION**

(43) Date of publication:
**09.06.2021 Bulletin 2021/23**

(51) Int Cl.:
***G01N 33/00*** (2006.01)     ***G08B 29/26*** (2006.01)

(21) Application number: **19213789.1**

(22) Date of filing: **05.12.2019**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>**KH MA MD TN**<br><br>(71) Applicant: **Sensirion AG**<br>**8712 Stäfa (CH)** | (72) Inventor: **Diether, Salomon**<br>**8712 Stäfa (CH)**<br><br>(74) Representative: **Toleti, Martin**<br>**E.Blum & Co. AG**<br>**Vorderberg 11**<br>**8044 Zürich (CH)**<br><br>Remarks:<br>Amended claims in accordance with Rule 137(2) EPC. |

(54) **SELF-TUNING PROCESSING OF ENVIRONMENTAL SENSOR DATA**

(57)     A method is provided for processing environmental sensor data comprising the following steps. One or more raw data values are received from an environmental sensor, an average value and a measure of dispersion are determined over a defined time period only from raw data values between a lower threshold and an upper threshold, and the lower threshold and the upper threshold are redefined depending on the average value and the measure of dispersion. The method may be used in an environmental sensor, e.g. a MOX sensor or a VOC sensor, and implemented as a computer program.

Fig. 3

**Description**

Technical Field

A method for processing environmental sensor data as well as a corresponding computer program and a corresponding environmental sensor are provided.

Background Art

Sensors are commonly used to measure physical quantities of the environment, e.g. a concentration of a gas or of volatile organic compounds (VOC), humidity or temperature. Such sensors are denoted as environmental sensors and may in particular be metal oxide (MOX) sensors, e.g. for measuring a gas concentration. The environmental sensor transforms the measured physical quantity to raw data values, e.g. a voltage or a number of ticks, measured at consecutive points in time.

Environmental sensor data may be used to perform "state estimation", i.e. an estimation of statistical quantities from the raw data values, e.g. a mean, a variance or a standard deviation. Such state estimation is typically used for VOC content, in particular for classifying an environment according to its VOC content or for providing a VOC level classification.

In this context, a common problem is the treatment of "outliers", an outlier being defined as a raw data value that differs significantly from other raw data values. A source of error is e.g. an error within the sensor. In particular an outlier may be defined to differ by one or two standard deviations from the average of other raw data values.

A known solution is "gating", i.e. the exclusion of unlikely data from state estimation. Gating is usually performed as "naive gating" with "hard" lower and upper thresholds which are constant over time: Raw data values are excluded from state estimation if they are below the lower threshold or above the upper threshold. An example for such upper/lower threshold is the mean plus/minus the standard deviation.

However, such naive gating has the disadvantage that it leads to incorrect results when the overall state of the environment changes significantly, e.g. by more than one standard deviation, in particular when the environmental sensor is transferred to a different place as is the case in mobile devices.

It is hence an object of the present invention to provide a method for processing environmental sensor data that yields robust and reliable results and preferably an intuitive user experience, in particular under significant changes of the environmental state.

Disclosure of the Invention

This problem is solved by a method for processing environmental sensor data which comprises the following steps: receiving from the environmental sensor one or more raw data values; determining an average value and a measure of dispersion over a defined time period only from raw data values between a lower threshold and an upper threshold; and redefining the lower threshold and the upper threshold depending on the average value and the measure of dispersion.

The above steps may be iterated for a continuous processing in time. In a discrete system, the steps may be implemented within a loop in a computer program. Such method may be called "self-tuning gating" since the lower threshold and the upper threshold are adaptable over time depending on the re-determined average value and the re-determined measure of dispersion. The self-tuning gating has the advantage that gradual changes of the environmental state are possible while the processing method still yields reliable results.

An environmental sensor is considered a sensor used to measure physical quantities of the environment, e.g. a concentration of a gas or of volatile organic compounds (VOC) in the environment, humidity of the environment or temperature of the environment. Most of the times the environment is ambient air. The environment of the sensor preferably is considered an environment of a device or a housing the sensor is comprised in, preferably arranged with a sufficient coupling of the sensor to the environment of the device or housing. Any such sensor is referred to as environmental sensor and may in particular be a metal oxide (MOX) sensor, e.g. for measuring a gas concentration. The environmental sensor transforms the measured physical quantity to raw data values, e.g. a voltage or a number of ticks, measured at consecutive points in time. Any such raw data value may represent the measured physical quantity at the measuring point in time, also referred to as discrete time step. The physical quantity at the measuring step in time can be derived from the raw data value. The term raw data value does not exclude a pre-processing of the signal actually originating from the sensitive element of the environmental sensor. Such pre-processing may include one or more of linearization, compensation e.g. for temperature or other impact, analogue-to digital conversion if applicable, etc. In a preferred embodiment, the environmental sensor is embodied as a chip comprising a semiconductor, wherein the chip may at the same time comprise pre-processing functions in form of an integrated circuit.

In one embodiment, it is assumed that at least in a steady state of the sensor system, a single raw data value is provided by the sensor at one discrete point in time, and hence is received by the processing entity preferably executing the method steps during operation. Such raw data value is evaluated as to its value / amplitude. For such evaluation, at least two thresholds are provided, i.e. a lower threshold and an upper threshold. For example, under the assumption that the raw data values of the environmental sensor can only take values in a positive range, the lower and the upper thresholds are defined in such positive range, too. In particular, the lower threshold exceeds the lower bound of the range of raw data values, which lower bound may, for example, be zero while the upper threshold is less than the upper bound of the range of the possible raw data values.

A raw data value outside of the range between the lower threshold and the upper threshold preferably is considered as an event on the one hand, however, as statistical outlier on the other hand. In different words, such raw data value outside the inter-threshold-range indicates a situation in the environment that may be noticeable in a sense that a notification may be issued and / or that the raw data value itself or a derivative thereof may be issued, e.g. to an operator or to a person present in the ambient of the environment sensor. The notification may take the form of a warning or an alert, e.g. an optical and/or an acoustic and/or a haptic warning, issued e.g. by the device containing the environmental sensor or transmitted elsewhere, e.g. to a remote operator or monitoring station. On the other hand, such event is preferentially considered not to impact the statistics, i.e. the determination of the average value and the measure of dispersion.

In case the environmental sensor is a gas sensor to detect one or more gases detrimental to humans above critical concentrations, the thresholds may be such that when the upper threshold is exceeded, a critical concentration of the gaseous component measured is reached or exceeded. Other applications may also be interested in the falling of a / the raw data values below the lower threshold. E.g., this may be the case in a room the (bad) air of which is impacted e.g. by opening a window and letting fresh air pour in.

However, fixed thresholds in form of constant thresholds over time may prove vulnerable in that isolated "outliers" or even permanent "outliers" exceeding the threshold may either occur rarely, or may not have the origin in a corresponding high concentration but rather in error scenarios or a change in a reference value in comparison to which reference value a measured concentration value is to be set. Accordingly, an outlier is defined as a raw data value that differs significantly from other raw data values.

Instead, it is presently proposed that the thresholds are allowed to vary over time in order to adjust to new reference scenarios. In one example, such a measurement scenario in the context of gas sensing may include measuring the concentration of a specific gaseous component in a room, where only little of the gaseous component is present as typical background concentration. In a different room a different background concentration of this gaseous component may prevail and may be considered a different measurement scenario. The environmental sensor may be moved from the first room into the second room during lifetime. While in the first scenario, only little deviations from the background concentration may be tolerable, in the second room, for whatever reason, larger deviations from the substantial background concentration may be tolerable.

For satisfying such changes in scenarios / measurement environments, and / or addressing different reasons for outliers, the thresholds are embodied dependent from raw data values earlier in time, and hence from the statistics of the raw data values of the past. Accordingly, an average value of previous raw data values is determined as well as a measure of dispersion. Preferably, both the average value and the measure of dispersion are statistical values / parameters, wherein the measure of dispersion indicates the extent to which a distribution (presently of raw data values) is stretched or squeezed around the average value. The average value and the measure of dispersion are preferably determined for the same defined period in time in the past, e.g. for the last ten hours, or e.g. for the last 24 hours. In an embodiment, the defined time period is at least one hour, preferably at least one day, preferably between one and three days. In specific scenarios, the defined period in time may be different for building the moving average and the building of the moving measure of dispersion. The defined time period determines from how far back in the past raw data values are taken into account in the determination of the present average value and measure of dispersion. In one embodiment, the raw values of the past may be weighted, e.g. the most recent ones with a bigger weight than the older ones. Hence, older raw data values contribute less to the respective statistical parameter than younger raw data values.

As time passes and new raw data values arrive from the environmental sensor, the average value and the measure of dispersion are updated, preferably at each discrete point in time a new raw data value arrives. In one embodiment, it is assumed that one raw data value is received per discrete time step, such that the subsequent determination of the average value and the measure of dispersion is dependent on this raw data value received for the corresponding time step. In response to the determination of the average value and the measure of dispersion the lower threshold and the upper threshold are redefined for this time step dependent on the average value and the measure of dispersion determined for the corresponding time step. Such calculation is repeated for each time step and preferably triggered by each new raw data value received.

In a preferred embodiment, the average value and / or the measure of dispersion are determined recursively. Preferably, both variables are determined recursively. In particular, the average value and / or the measure of dispersion are determined by exponential smoothing functions each. For example, as to the average value, the average value $av_t$ is determined by

$$av_t = \alpha * av_{t-1} + (1-\alpha) * rdv_t$$

with $av_t$ as average value at discrete time step t, $av_{t-1}$ as average value at discrete time step t-1, $\alpha$ as smoothing factor, and $rdv_t$ as raw data value received at discrete time step t.

The measure of dispersion preferably is determined by

$$\sigma_t = \sqrt{\alpha * (\sigma^2_{t-1} + (1-\alpha)(rdv_t - av_{t-1})^2)}$$

with $\sigma_t$ as standard deviation at discrete time step t, $\sigma_{t-1}$ as standard deviation at discrete time step t-1, $av_{t-1}$ as average value at discrete time step t-1, $\alpha$ as smoothing factor, and $rdv_t$ as raw data value received at discrete time step t.

The smoothing factor $\alpha$ determines, how much weight is assigned to the raw data values of the past, which may be in the range of e.g. $\alpha$ = [0.8, 1], while each new data value is assigned less weight, e.g. $1-\alpha$ = [0.0, 0.2]. Such approach is easy to implement in discrete systems and saves computing power.

However, in a different embodiment, both of or one of average value and measure of dispersion is determined non-recursively.

Preferably, the average value corresponds to an arithmetic mean of the raw data values over the defined period in time, while the measure of dispersion preferably corresponds to the standard deviation of the raw data values in the defined period in time, or e.g. two time of the standard deviation. In such case, the underlying assumption is that the raw data values follow a normal distribution.

In a different embodiment the average value is taken to be the median of the raw data values, which may have the advantage to be independent of an assumed distribution of the raw data values and in particular less prone to distortion due to outliers. Preferably in such embodiment, the measure of dispersion is an interquartile range or a range between a 10th and a 90th percentile.

However, the term average value shall also encompass other methods for determining what can be expressed as a typical value, i.e. a value the raw data values typically take over time. Other methods for determining such average value shall be comprised in the term average value.

In turn, the upper and the lower thresholds are preferably redefined in each discrete time step, as are the average value and the measure of dispersion, i.e. in response to the receipt of each new raw data value. In particular the lower and the upper threshold are redefined dependent from the just calculated updates of the moving average value and the moving measure of dispersion.

According one choice of definition, the lower threshold is the average value minus the measure of dispersion, and the upper threshold is the average value plus the measure of dispersion. For the case of the average value being the arithmetic mean and the measure of dispersion being one or two standard deviations, this means that approximately 68.3% or 95.4%, respectively, of the raw data is used for determining a new average value and a new measure of dispersion - and consequently for adapting the lower threshold and the upper threshold - while 31.7% or 4.6%, respectively, of the raw data values are disregarded under the assumption of a normal distribution. Such values have proven to yield plausible results. In particular such choice of definition enables an intuitive user experience.

Hence, in an embodiment, a preferred sequence of steps is to receive a raw data value, and in response to the receiving of the raw data value, determine if the raw data value is between the lower threshold and the upper threshold. The lower threshold and the upper threshold may, in a steady state of the sensor system, be calculated in a previous step prior to receiving the above raw data value. At the beginning of a measurement, initial values may be set for the thresholds and / or the average value and the measure of dispersion as will be described on more detail below.

In response to determining if the raw data value received at time $t_0$ is between the upper threshold and the lower threshold, an average value and a measure of dispersion is determined. Given the steady state of the system, the average value and the measure for dispersion were also determined in the previous step at time $t_{-1}$, and for an assumed period in time of e.g. $t_{-1}-x*\Delta t$, with $\Delta t$ being the interval between two discrete points in time, and x being a predefined constant. In one example, let x be 100 and the previous average value was determined by making use of the raw data values between $[t_{-101}; t_{-1}]$ including the boundary values. In contrast, at the present point in time to the average value is now determined over all the raw data values in the interval of $[t-100; t_0]$ including the boundary values. However, in the present determination of the average value, raw data values not being in the range between the lower threshold and the upper threshold are discarded, i.e. they do not contribute to the forming of the average value when received within this period in time. In a different notation, a new raw data value contributes to the forming of the moving average according to the function:

1 if within the range between the lower and the upper threshold;
0 if outside the range between the lower and the upper threshold.

Note that in view of the thresholds varying, the inclusion or exclusion of a raw data value always is dependent relative to the lower and the upper threshold at that point in time, i.e. dependent on the values of the lower and the upper threshold valid at the point in time the respective raw data value arrives. The same is true in the determination of the measure of dispersion.

Accordingly, the average value and the measure of dispersion preferably are moving over a constant period back in time which period / window progresses as time progresses and new raw data values are available. However, it is preferred that only those raw data values within such window contribute to the calculation of the average that were, at the point in time of their evaluation versus the then valid lower and upper threshold found to be within the range between the then valid lower and upper threshold. Raw data values outside this range do not make it into the calculation of the average value neither at the point in time of receipt, nor in subsequently calculated average values, as long as the raw data values belong to the sliding window. The same is true for the measure of dispersion.

In case of the above embodiment of recursive computing of the average value, the updated average value $av_t$ is determined by

$$av_t = \alpha * av_{t-1} + (1-\alpha) * rdv_t$$

in case of the new raw data value $rdv_t$ is within the upper and the lower threshold. In case the new raw data value is outside the upper and the lower threshold, the updated average value $av_t$ is set to the previous average value:

$$av_t = av_{t-1}$$

In case of a non-recursive computing of the average value, the updated average value $av_t$ at time t is determined by

$$av_t = 1/N \sum_{i=-N+1}^{t} \psi i \; rdvi$$

with N being the defined time period / window size, and $\psi_i$ being an operator of value 1 if the corresponding raw data value rdvi is within the thresholds, and is of value 0 if the corresponding raw data value $rdv_i$ is outside the thresholds.

Once the average value and the measure of dispersion are re-defined in response to a new raw data value received, the thresholds are redefined dependent on such re-defined average value and such re-defined measure of dispersion. Accordingly, in response to the receipt of a new raw data value, first the stochastic measures are updated, then the thresholds are updated in dependency from the updated stochastic measures.

It is advantageous that the method comprises the step of determining normalized data values from the raw data values depending on the average value and / or the measure of dispersion. Such method may additionally comprise the step of outputting the normalized data values. The determination of normalized data values may e.g. be implemented by the following two steps: subtracting the average value from the raw data value and dividing the resulting difference by the measure of dispersion. Such determination in particular leads to a dimensionless number. In general, the normalized data values represent relative values such that changes of the underlying physical quantity are easily appreciated. In particular, this leads to a good user experience since users are often interested in changes over time but not in the absolute value, e.g. in the case of a VOC or gas concentration. Preferably, such normalized data values are output, and preferably all normalized data values are output irrespective if above the upper threshold, below the lower threshold, or between the lower and upper threshold. Preferably, per each time step the normalized data value is determined a new from the corresponding received raw data value and the average value and measure of dispersion determined for the same time step, i.e. preferably based on the updated average value and measure of dispersion dependent on the new raw data value received.

Preferably, the method comprises the step of defining as an event, if the raw data value is not between the lower threshold and the upper threshold. In other words, an outlier as defined above is regarded as an event. This is useful since an outlier may on the one hand just come from an error or noise within the sensor, but on the other hand it may be due to an actual event, i.e. a significant change in the measured physical quantity, and hence be associated with a change in the environmental state. In particular a notification may be output in case of an event. Such notification may be useful in the case of an application monitoring the environmental state and e.g. detecting certain conditions, e.g. a VOC or gas concentration that is potentially detrimental to a person's health. In embodiments, the notification may be a signal transferred to a remote device, a sound or an alarm.

The method described above may further be detailed by the next preferred embodiments encompassing a disablement of the gating and / or a soft threshold, respectively. Both embodiments lead to a "robust self-tuning gating" as will become evident from the following description, and they may be applied separately or in combination.

In a first class of embodiments, the gating applied for the statistical calculations, i.e. average value and measure of dispersion, may not be appropriate in all specific scenarios. Hence, it is envisaged, that the gating is at least temporarily disabled, i.e. switched off. A trigger for switching off such gating is referred to as gating adaptation event since a temporary switching off of the gating allows the gating to adapt to changed environmental conditions, for example. In response to such gating adaptation event, any raw data value received, even if outside the lower and the upper threshold is included in the determination of the statistical parameters average value and measure of dispersion and contributes thereto. Hence, in the step of calculating the average value and measure of dispersion subsequent to the occurrence of the gating adaptation event, the then received raw data value/s contribute/s to the calculation of the average value and the measure of dispersion in any case irrespective of value. In case raw data values received prior to the gating adaptation event shall contribute to the determination of the statistical parameters - which will always be the case for the determination of the statistical parameters within the interval $[t_x ; t_x + T]$ with $t_x$ being the point in time of the gating adaptation event and T being the defined time period, i.e. the sliding window, - only raw data values within the lower and upper threshold may be accepted, while raw data values outside the threshold range remain disregarded. Hence, in a preferred embodiment, the inclusion of raw data values outside the thresholds in the determination of the average value and the measure of dispersion only start in response to the gating adaptation event, but does not have retroactive effect.

This approach is perfectly reflected in a recursive determination of the statistical parameters in which the previous average value / dispersion measure prior to the gating adaptation event is weighted and hence contributes to the determination of the first average value after the gating adaptation event such that outliers of the past beyond the thresholds remain excluded. However, preferably starting with the occurrence of the gating adaptation event, any new raw data values contribute to the calculation of the statistical parameters irrespective of their value.

In other words, between the gating adaptation event and a gating adaptation disengagement event at which the switching off of the gating is disabled, the average value and the measure of dispersion are preferably determined dependent on all raw data values received after the gating adaptation event even if not between the lower threshold and the upper threshold, as long as the defined time period exceeds an interval between the gating adaptation event and the gating adaptation disengagement event.

Hence, the average value and the measure of dispersion for a time step after the gating adaptation trigger is determined dependent on the raw data value received for the corresponding time step even if not between the lower threshold and the upper threshold. In particular, between the gating adaptation event and a gating adaptation disengagement event and as long as the defined time period exceeds an interval between the gating adaptation event and the gating adaptation disengagement event, the average value and the measure of dispersion are determined dependent on all raw data values even if not between the lower threshold and the upper threshold received on or after the gating adaptation event, and preferably are determined dependent on the last average value and the last measure of dispersion determined prior to the gating adaptation event.

By means of such disabling of the gating, the average value and the measure of dispersion are adapted to new scenarios representing different average levels and different levels of dispersion of the chemical concentration, for example. Hence, soon after disabling gating, raw data values which previously were considered as outliers then remain within the gating thresholds since the lower and the upper thresholds are adapted. In adapted environmental scenarios, it is further assumed that outliers now rather represent a new average level.

In a first preferred embodiment ("maximum gating ratio") of the gating adaptation event, the method comprises the step of determining, for a monitoring period, a ratio between a number of raw data values that are not between the lower threshold and the upper threshold and a total number of raw data values. Such ratio may also be regarded as a discarding rate. If the ratio becomes large, this means that many, e.g. more than 31.7% or 4.6%, respectively, of the raw data values, are regarded as outliers or events and not taken into account in the determination of the average value, the measure of dispersion and consequently in the redefinition of the lower threshold and the upper threshold.

As a counter-measure, the method preferably further comprises the adaptation of the average value and the measure of dispersion determination as laid out above.

Preferably, the gating adaptation disengagement event is set after a predefined time has passed from the gating adaptation event. Hence, the disengagement of the gating adaptation, i.e. the return to the gating, preferably is controlled in time, e.g. in the range between one minute, ten minutes and one hour.

Such method proves particularly useful if a sudden and significant change in the raw data values occurs, which may e.g. be due to a change in the environmental state. While a pure "self-tuning gating" method as described before may not show any values at all in such situation, the self-tuning gating with maximum gating ratio will continue to yield reasonable results. Hence the method is more robust and in particular leads to an improved user experience.

As a variant of the "maximum gating ratio", the method may comprise the following additional step: The gating adaptation event now is defined, if for a monitored period in time, no raw data value is between the lower threshold and the upper threshold. The method triggered by the gating adaptation disengagement event is the same as laid out in connection with the "maximum gating ratio".

In the second preferred embodiment ("soft threshold"), the method comprises the following steps: determining weights of a weighting function for the raw data values depending on the average value and / or the measure of dispersion; and applying the weights to the raw data values when determining the average value and the measure of dispersion. In particular this means that the influence of the raw data values is not the same and e.g. depends on their difference to the average value.

Such embodiment may be further detailed as follpws: The weights are defined largest at or around the average value. In particular the weights may increase monotonically for raw data values between the lower boundary of the raw data value range and the average value. Also the weights may decrease monotonically for raw data values between the average value and the upper boundary of the raw data value range.

In general, the described second preferred embodiment has the effect that a hard threshold can be avoided by imposing such soft threshold through the weighting function. In particular, this avoids the unwanted effect that of two neighbouring raw data values on opposite sides of the lower threshold or the upper threshold, one goes into the determination of the average value and the measure of dispersion, and consequently into the state estimation, whereas the other one does not. This unwanted situation e.g. has the effect that two sensors of the same type may after a while show totally different values when operating near the lower or upper threshold. The self-tuning gating with soft threshold avoids this effect, leads to reliable and robust sensor output and in particular to an intuitive user experience.

As stated at the beginning of this section, the method may further comprise iterating some or all of the described steps. Depending on the initial condition, there may be the following two embodiments, which may be referred to as "initial values" and as "initial training phase":

In the first embodiment, the method comprises the following steps: At the beginning, initial values for the lower threshold and the upper threshold are received, and then the above described steps are iterated. Preferably, the initial values for the lower threshold and the upper threshold are chosen such that a large fraction, in particular more than 50%, 75% or 90% fall between the lower threshold and the upper threshold. In particular, the first of the iterating steps of the first embodiment comprises receiving more than one raw data values in order to make sure that a reasonable measure of dispersion can be determined.

The second embodiment may be regarded as having first an initial training phase before the iteration. It comprises the following steps: At the beginning, initial raw data values are received from the environmental sensor, an average value and a measure of dispersion is only then determined from the initial raw data values, e.g. after receipt of the first 50 raw data values, and a lower threshold and an upper threshold are only then defined depending on the average value and the measure of dispersion. Then the above described steps are iterated. Preferably the initial raw data values correspond to a training time period, which may e.g. in the range between one minute, ten minutes, one hour and one day.

In a second aspect of the invention, a computer program comprises instructions which, when the program is executed by a processor, cause the processor to execute the steps of the method described above.

A third aspect of the invention relates to an environmental sensor which comprises a sensor and a processor adapted to execute the steps of the method described above. In particular the sensor may comprise a MOX sensor, e.g. for measuring a presence or concentration of a gas. The sensor may particularly comprise a VOC sensor.

Other advantageous embodiments are listed in the dependent claims as well as in the description below.

Brief Description of the Drawings

The invention will be better understood and objects other than those set forth above will become apparent from the following detailed description thereof. Such description makes reference to the annexed drawings, wherein:

Fig. 1 shows a time series of raw data and of different thresholds determined from the raw data in a conventional way and, respectively, in a method with gating according to an embodiment of the invention;

Fig. 2 shows a flow chart of a method for processing environmental sensor data according to an embodiment of the invention;

Fig. 3 shows a time series of raw data and of different thresholds applying "normal gating" and "robust gating", respectively, according to embodiments of the invention;

Fig. 4 shows a flow chart of a method for processing environmental sensor data applying robust gating according to an embodiment of the invention;

Figs. 5a and 5b show time series of two raw data signals each and of corresponding output signals when gating with a "hard threshold" is applied (Fig. 5a) and when a "soft threshold" is applied (Fig. 5b) according to embodiments of the invention;

Fig. 6 shows a weighting function with weights depending on the raw signal value for use in a method for processing environmental sensor data applying gating with a "soft threshold" according to an embodiment of the invention.

Modes for Carrying Out the Invention

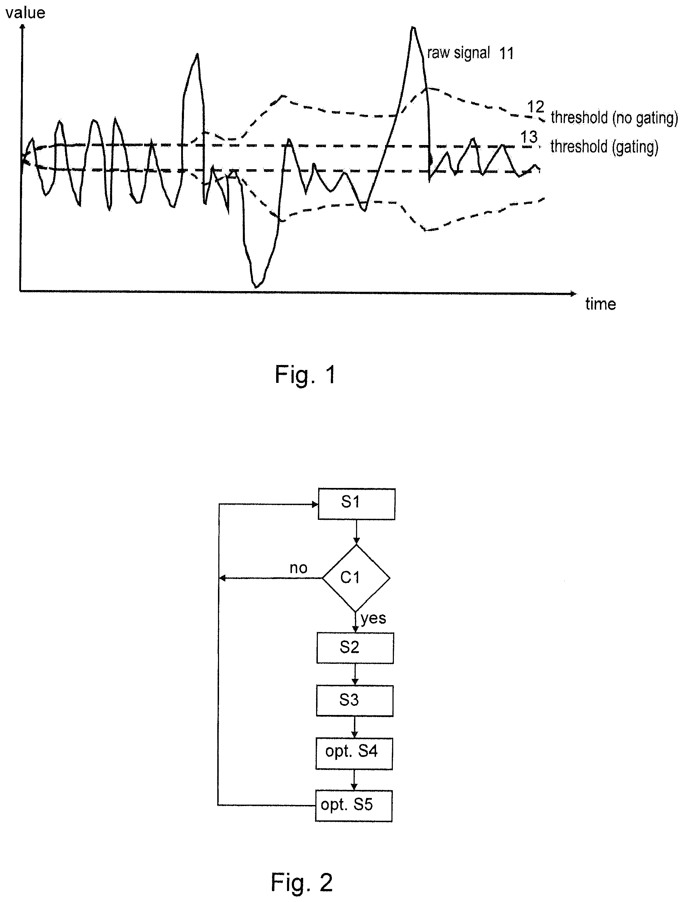

Fig. 1 shows an example time series of a raw signal 11, i.e. raw data values, as measured by an environmental sensor and optionally pre-processed, e.g. by a calibration algorithm. In the example time series, the raw signal 11 fluctuates around some constant value (not shown), while some of the raw data values are far off, i.e. significantly higher or lower than the constant value. Such significantly deviating raw data values, which are e.g. deviating from a mean / average value of the raw signal by more than one or more than two standard deviations, are denoted as outliers.

Further, Fig. 1 shows a lower threshold and an upper threshold 12 as determined without any gating. The threshold value 12 at a certain time is calculated as the mean value plus (for the upper threshold), and respectively minus (for the lower threshold), the standard deviation of all raw data values over a defined time period before the certain time. It is a known problem that the threshold 12 heavily depends on outliers, i.e. a significantly deviating raw data values strongly influences the threshold 12 as shown in Fig. 1.

It is proposed to apply a gating algorithm when determining the threshold, the result of which is shown as lower threshold and upper threshold 13 in Fig. 1. Gating means that in the determination of the lower and upper threshold 13 at a certain time only raw data values are taken into account which are between an earlier determined lower and upper threshold. This means that outliers are excluded from the determination of the mean and the standard deviation and consequently of the new lower and upper threshold. While both thresholds 12 and 13 adapt to the raw data values over time, i.e. they are not constant in time, the threshold 13 with "self-tuning" gating is more robust against outliers. Such threshold 13 with self-tuning gating may be obtained by the method depicted in Fig. 2.

Fig. 2 shows a flow chart of a method for processing environmental sensor data according to an embodiment of the invention. The method comprises the steps S1, S2 and S3 as well as optional steps S4 and S5. The steps may be iterated and e.g. be implemented as a loop in a computer program. In step S1, a "new" raw data value rdv is received from an environmental sensor, such as a MOX gas sensor or a VOC sensor. With condition C1, it is checked whether the new raw data value rdv falls within the interval between the lower threshold lt and the upper threshold ut. Condition C1 presupposes that a lower and an upper threshold are given, e.g. from an earlier determination step or as initial threshold values. Only if the new raw data values rdv fulfills the criterion lt <= rdv <= ut, the processing proceeds with step S2; otherwise it jumps back to step S1. Hence condition C1 may be seen as the "gate" in the gating algorithm.

In step S2, the arithmetic mean av and the standard deviation $\sigma$ of the raw data values that fulfil condition C1 are determined. In the example of Fig. 1, the mean and the standard deviation are calculated over the defined time period, e.g. an hour or a day. When the method of Fig. 2 is iterated, this amounts to a moving window for which the mean and the standard deviation are determined, preferably in a recursive way, in which the updated average value av is determined by

$$av = \alpha * av_{-1} + (1-\alpha) * rdv$$

in case of the raw data value $rdv_t$ is equal to or within the upper and the lower threshold ut and lt, i.e. "C1=yes". In case the raw data value is outside the upper and the lower threshold ut and lt, i.e. "C1 = no", the updated average value $av_t$ is set to the previous average value: $av = av_{-1}$

In general, the mean may be replaced by any average value, e.g. a median which typically is a more robust measure of average than the arithmetic mean, and the standard deviation may be replaced by any measure of dispersion, e.g. an interquartile range. In a specific embodiment, the average value and the measure of dispersion may be calculated recursively, e.g. by exponential smoothing functions as described above, which saves computing power and storage space.

In step S3, the lower threshold lt and the upper threshold ut are redefined depending on the average value and the measure of dispersion determined in step S2. This may be done by the relations lt = av - no and ut = av + no, wherein n is a constant factor which is typically in the range between 1 and 3, e.g. 2. Alternatively, the lower threshold lt may be chosen to be a percentile, e.g. the 10th or 25th percentile, of the raw data, and the upper threshold ut to be a higher percentile, e.g. the 90th or 75th percentile.

The lower threshold lt and the upper threshold ut redefined in step S3 replace their former values. Thus they are taken into account in the gate condition C1 in the next iteration of the loop depicted in Fig. 2.

In a variant, the method of Fig. 2 also comprises one or both of the optional steps S4 and S5. In step S4, a normalized data value ndv is determined from the raw data value rdv. A typical normalization is the subtraction of the average value as ndv = rdv - av. Alternatively, the normalized data value may additionally depend on the measure of dispersion, e.g. as ndv = (rdv - av) / $\sigma$. Such normalized data value is useful in that it better shows relative changes in the measured quantity than the raw data value. Thus the normalized data values are naturally suited as output quantities, e.g. for a rapid perception of relevant changes by the user and for a good user experience.

Accordingly, the normalized data value ndv is output in optional step S5. The output may be implemented as numerical value which is stored or transferred to a remote system via an interface. Or the normalized data value ndv may be output by means of a display, e.g. as graph showing the normalized data values over time.

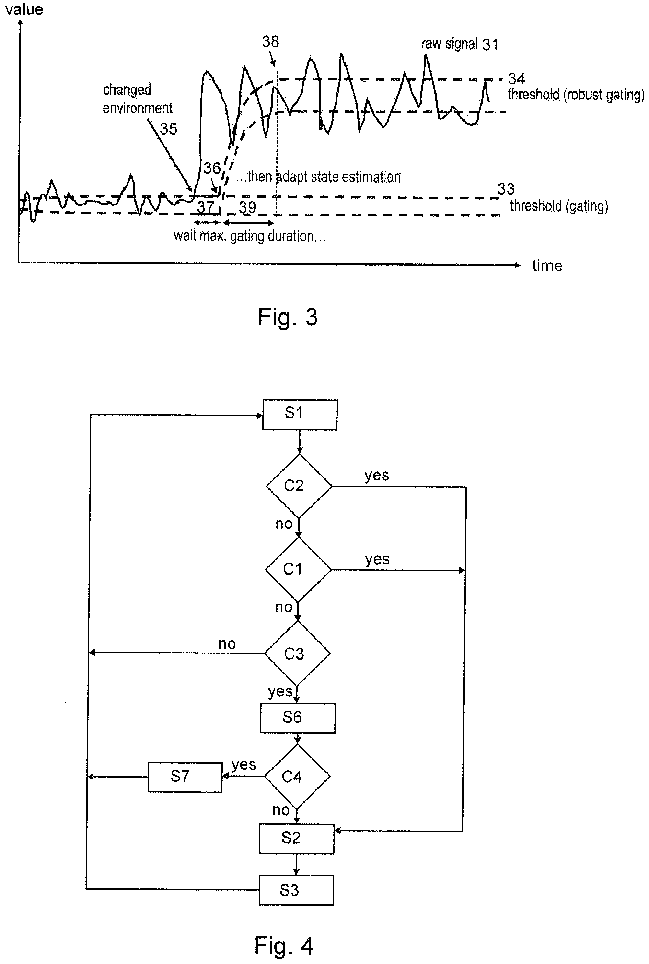

Fig. 3 again shows a time series of a raw signal 31, i.e. a plurality of raw data values rdv. In this case, the raw signal 31 shows an abrupt increase at point 35, which is due to a change of environment, e.g. because the environmental sensor is transferred to another place or because a gas concentration at the environmental sensor changes abruptly. For the present purpose, an abrupt change is defined as a change in raw data values by more than the measure of dispersion, e.g. by more than one standard deviation.

Further, Fig. 3 shows lower and upper thresholds 33 as obtained by the above described method of Fig. 2. After point 35, at which time the environment changes, all raw data values fall outside the range between the lower and upper threshold 33. This means that none of the later raw data values passes the gate condition C1, and that the above method never reaches steps S2 and S3. Hence the average value av and the measure of dispersion $\sigma$ are not re-determined, and the lower and upper threshold 33 are not redefined, such that all later raw data values are treated as outliers.

Such behaviour is undesired, especially if the later raw data values represent a new environmental state which is stable at least for some time, e.g. 1 min, 10 min or 1 h. In the case of normalized data values and an output according to steps S4 and S5, such situation leads to useless results. Either the normalization as described above works insufficiently and yields unreasonable results or, even worse, the outliers may even not be displayed at all.

As a remedy for such situation, a "robust gating" is proposed which leads to the lower and upper thresholds 34 in Fig. 3. In robust gating, the concept of a gating adaptation event 36 is introduced. At the gating adaptation event 36, the gating is suspended, i.e. condition C1 is removed from the method of Fig. 2, and all raw data values rdv are taken into account for the determination of the average value av and the measure of dispersion $\sigma$ in step S2. In this way, the lower and upper thresholds 34 will adapt to the raw data values on the different level due to the changed environment, but only after the gating adaptation event 36. Hence robust gating facilitates reliable sensor readings and state estimation even under abruptly changing environmental conditions.

One way of implementing the gating adaptation event 36 is looking at the time period for which no raw data value rdv fulfilled the gating criterion C1. If this time period exceeds a monitoring time period 37, e.g. 1 min, 10 min or an hour, then the gating adaptation event 36 is triggered.

An embodiment of a method for processing environmental sensor data applying robust gating is shown in the flow chart of Fig. 4. Step S1 and condition C1 are analogous as in the method of Fig. 2. Criterion C2 represents the check whether gating adaptation is enabled. If the gating adaptation is enabled, i.e. there has been a gating adaptation event, a raw data value rdv after being received in step S1 directly goes into the re-determination of the average value av and the measure of dispersion $\sigma$ in step S2, regardless of whether it would pass the gating criterion C1.

On the other hand, if the gating adaptation is not enabled, it is checked whether the raw data value rdv falls within the interval between the lower threshold and the upper threshold in criterion C1. If yes, rdv is directly passed to step S2 and contributes to the re-determination of av and $\sigma$. If no, it is checked with criterion C3 whether the conditions for a gating adaptation event are fulfilled. If the conditions for the gating adaptation event are fulfilled in C3, the gating adaptation event is triggered in step S6. This means that in step S6 the gating adaptation is enabled. If the conditions for the gating adaptation event are not fulfilled in C3, the raw data value rdv is not taken into account in the re-determination of av and $\sigma$, and the processing starts again with step S1, i.e. receiving a new raw data value.

As shown in the embodiment of Fig. 3, the condition for the presence of a gating adaptation event in criterion C3 may be formulated as: Does the time period since the last raw data value fulfilled criterion C1 exceed the monitoring time period? Alternatively, the gating adaptation criterion C3 may comprise the determination of a ratio between a number of raw data values not fulfilling the gating criterion C1 and a total number of raw data values during the monitoring time period. In that case, the gating adaptation event is triggered if the ratio is larger than a maximum ratio, e.g. 10%, 25% or 33%.

As it is not reasonable to take into account all raw data values for an infinite time after one gating adaptation event is triggered in step S6, it is useful to define a gating adaptation disengagement event 38 as shown in Fig. 3. The gating adaptation disengagement event 38 causes the data processing to take the normal "gated" route, i.e. after such event 38 newly received raw data values contribute to the re-determination of the average value, the measure of dispersion and the lower and upper thresholds only if they fulfil the gating criterion C1. In the embodiment of Fig. 3, the gating adaptation disengagement event 38 is triggered when a predefined period in time 39, e.g. in the range of 10 min to an hour, has passed since the last gating adaptation event 36.

In terms of the method depicted in Fig. 4, the gating adaptation disengagement event 38 may be represented as gating adaptation disengagement criterion C4, e.g. as a check whether the time since the last gating adaptation event 36 exceeds the predefined period 39. Alternatively, the gating adaptation disengagement criterion C4 may be formulated as whether a certain number of raw data values received since the last gating adaptation event 36 have passed the gating criterion C1.

If the outcome of the check of criterion C4 is positive, the gating adaptation disengagement event 38 is triggered, the gating adaptation is disabled in step S7, and the processing restarts with step S1. If the outcome of the check of criterion C4 is negative, the present received raw data value is taken into account in the re-determination of av and $\sigma$ in step S2 and in the redefinition of the lower and upper thresholds in step S3. Details for steps S2 and S3 have been described earlier.

In general, the method of Fig. 4 may additionally comprise steps S4 and S5 as described above. Also a different ordering of the steps as well as variations in the implementation are possible.

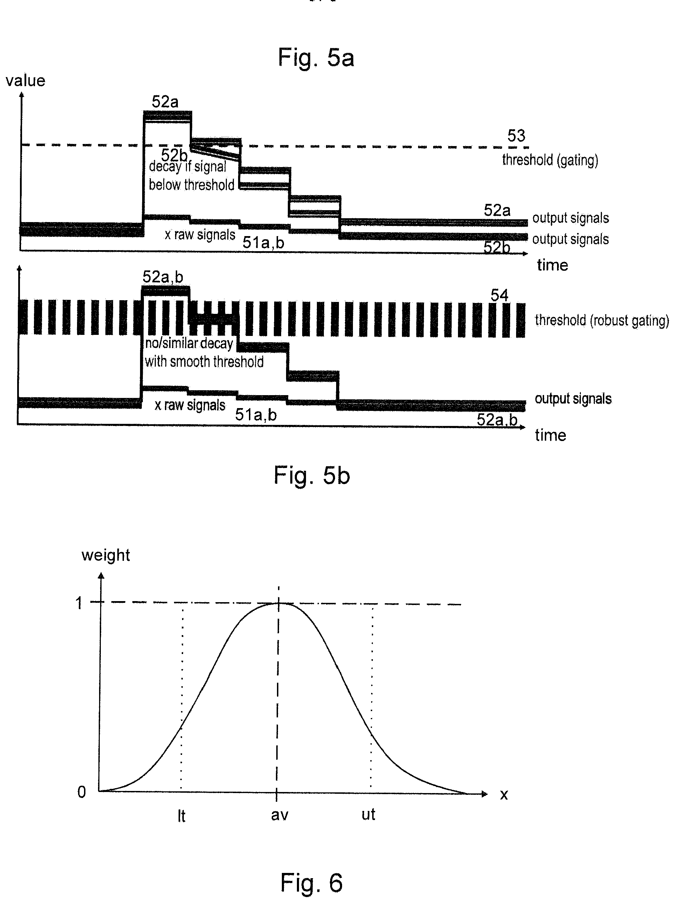

Figs. 5a, 5b and 6 show embodiments of a different aspect of a method applying "robust gating" which may be combined with the earlier described aspects. Fig. 5a depicts a time series of an undesirable situation what may occur in terms of output signals 52a, 52b in case of two raw signals 51a, 51b that slightly differ from each other. The raw signals 51a, 51b may be measured by two different environmental sensors of the same type. Both sensors are assumed to have a same current upper threshold 53. However, the raw data values of raw signal 51b measured by sensor B are slightly, e.g. by 1% or by 5%, lower than the ones of raw signal 51a measured by sensor A.

In case one or more raw data values of raw signal 51a are above the upper threshold 53, i.e. they do not pass the gating criterion C1, they do not contribute to the re-determination of av, $\sigma$ and the thresholds. Hence the threshold 53 for sensor A is not adapted. Raw data values measured by sensor B at the same time may, however, fall below the threshold 53 and hence pass the gating criterion C1. Thus av, $\sigma$ and the threshold for sensor B will be adapted on the basis of these raw data values. In that case, the average values av, the measures of dispersion $\sigma$ and the thresholds for sensor A and sensor B will begin to differ, and they will also continue to differ as long as no reset is performed.

Since the determination of the output signals 52a, 52b may depend on the present average value av and measure of dispersion a, see above in the context of normalized data, also the output signals 52a, 52b of the sensors A and B will differ. Such situation leads to non-intuitive readings of the two sensors and to a suboptimal user experience.

As a solution to such issue, a method for processing environmental sensor data with gating is proposed wherein the gating comprises a "soft" instead of a "hard" threshold. Fig. 5b shows the same time series of raw signals 51a, 51b of the sensors A and B as in Fig. 5a. However, instead of the hard threshold 53 of Fig. 5a, a soft threshold 54 is applied in Fig. 5b. Such soft threshold may be implemented as a weighting function, e.g. as shown in Fig. 6. Instead of applying the gating criterion C1 in the form of the check of lt < rdv < ut, raw data values rdv are weighted for the determination of the average value av and the measure of dispersion $\sigma$. Preferably raw data values near the previously defined average av have higher weights than raw data values further away from av, see e.g. Fig. 6.

As shown in Fig. 6, it is beneficial that the weighting function of a soft threshold is a continuous, in particular a smooth, function. Preferably, the weighting function tends to zero for raw data values far from the average value av. The weighting function may e.g. take the form of a Gaussian function around av and with the measure of dispersion $\sigma$ as standard deviation. For comparison, Fig. 6 shows as dotted line a weighting function that corresponds to the hard threshold of criterion C1 described earlier.

In general, the soft threshold with a weighting function has the effect that neighbouring raw data values, i.e. differing by 0 to 5%, get similar weights, i.e. differing by 0 to 5%. Hence in Fig. 5b, the output signals 52a, 52b, which are e.g. normalized versions of the corresponding raw signals 51a and 51b, respectively, differ only slightly. Such output is robust and reliable irrespective of the value range of the raw signals. Moreover it yields a desired user experience, in particular if two sensors of the same type are e.g. placed next to each other and measure the same environmental conditions.

**Claims**

1. Method for processing environmental sensor data, comprising the steps of

   - receiving from an environmental sensor one or more raw data values (11),
   - determining an average value and a measure of dispersion over a defined time period only from raw data values between a lower threshold and an upper threshold (13),
   - redefining the lower threshold and the upper threshold (13) depending on the average value and the measure of dispersion.

2. Method according to claim 1,
   wherein one of the raw data values is received per discrete time step, wherein the average value and the measure of dispersion are determined anew per time step dependent on the raw data value received at the corresponding time step, wherein the lower threshold and the upper threshold (13) are redefined anew per time step dependent on the average value and the measure of dispersion determined for the corresponding time step,

in particular wherein the lower threshold is the average value minus the measure of dispersion,
in particular wherein the upper threshold (13) is the average value plus the measure of dispersion,
in particular wherein the measure of dispersion corresponds to one or two times a standard deviation of the raw data values, and/or
in particular wherein the average value corresponds to an arithmetic mean of the raw data values, and/or
in particular wherein the defined time period is at least one hour, preferably at least one day, preferably between one and three days.

3. Method according to any of the preceding claims, additionally comprising the steps of

- determining normalized data values from the raw data values depending on the average value and/or the measure of dispersion, and
- outputting the normalized data values,
- preferably determining the normalized data value anew per time step from the corresponding raw data value depending on the average value and/or the measure of dispersion determined for the corresponding time step.

4. Method according to any of the preceding claims,
wherein the average value and the measure of dispersion are determined recursively each,
in particular wherein the average value and the measure of dispersion are determined by exponential smoothing functions each,
in particular wherein the average value is determined by $av_t = \alpha * av_{t-1} + (1-\alpha) * rdv_t$
with avt as average value at time t, $av_{t-1}$ as average value at time t-1, $\alpha$ as smoothing factor, and $rdv_t$ as raw data value received at time t,
in particular wherein the measure of dispersion is determined by

$$\sigma_t = \sqrt{\alpha * (\sigma^2_{t-1} + (1-\alpha)(rdv_t - av_{t-1})^2)}$$

with $\sigma_t$ as standard deviation at discrete time step t, $\sigma_{t-1}$ as standard deviation at discrete time step t-1, $av_{t-1}$ as average value at discrete time step t-1, $\alpha$ as smoothing factor, and $rdv_t$ as raw data value received at discrete time step t.

5. Method according to any of the preceding claims, additionally comprising the steps of

- in response to a gating adaptation event (36)
determining the average value and the measure of dispersion dependent on the raw data value received for the corresponding time step even if not between the lower threshold and the upper threshold,
in particular, between the gating adaptation event and a gating adaptation disengagement event (38) and as long as the defined time period exceeds an interval between the gating adaptation event (36) and the gating adaptation disengagement event (38), determining the average value and the measure of dispersion dependent on all raw data values even if not between the lower threshold and the upper threshold received on or after the gating adaptation event (36), and preferably dependent on the last average value and the last measure of dispersion determined prior to the gating adaptation event (36).

6. Method according to claim 5,

- determining for a monitoring time period (37) a ratio between a number of raw data values that are not between the lower threshold and the upper threshold and a total number of raw data values, and
- setting the gating adaptation event (36) if the ratio is larger than a maximum ratio.

7. Method according to claim 5,

- setting the gating adaptation event (36) if for a monitoring time period (37), no raw data value is between the lower threshold and the upper threshold.

8. Method according to any of the preceding claims 5 to 7,

- setting the gating adaptation disengagement event (38) after a predefined period in time (39) since the gating adaptation event (36),

- in response to the gating adaptation disengagement event (38)
determining the average value and the measure of dispersion dependent on the raw data value received for the corresponding time step only if between the lower threshold and the upper threshold,
in particular, after the gating adaptation disengagement event (38), determining the average value and the measure of dispersion per time step dependent only from the raw data values between the lower threshold and the upper threshold and received after the gating adaptation disengagement event (38), and preferably dependent on the last average value and the last measure of dispersion determined prior to the gating adaptation disengagement event (38).

9. Method according to any of the preceding claims, additionally comprising the steps of

- determining weights of a weighting function for the raw data values depending on the average value and the measure of dispersion,
- applying the weights to the raw data values when determining the average value and the measure of dispersion.

10. Method according to claim 9,
wherein the weights are largest at or around the average value, and
in particular wherein the weights increase monotonically for raw data values between zero and the average value, and/or
in particular wherein the weights decrease monotonically for raw data values between the average value and infinity.

11. Method for processing environmental sensor data according to any of the preceding claims, comprising the steps of

- at the beginning, receiving initial values for the lower threshold and the upper threshold,
- iterating the steps of the method according to any of the preceding claims.

12. Method for processing environmental sensor data according to any of the preceding claims, comprising the steps of

- at the beginning, receiving from the environmental sensor initial raw data values,
- determining an average value and a measure of dispersion from the initial raw data values,
- defining a lower threshold and an upper threshold depending on the average value and the measure of dispersion,
- iterating the steps of the method according to any of claims 1 to 10.

13. Method for processing environmental sensor data according to any of the preceding claims, comprising the steps of
in response to the receiving of the raw data value, determining if the received raw data value is between the lower threshold and the upper threshold,
in response to determining if the received raw data value is between the lower threshold and the upper threshold determining the average value and the measure of dispersion over the defined time period only from raw data values between the lower threshold and the upper threshold thereby including the received raw data value only if between the lower threshold and the upper threshold, thereby excluding the received raw data value from the determination of the average value and the measure of dispersion if not between the lower threshold and the upper threshold,
in response to determining the average value and the measure of dispersion redefining the lower threshold and the upper threshold depending on the determined average value and the determined measure of dispersion.

14. A computer program comprising instructions which, when the program is executed by a processor, cause the processor to execute the steps of the method according to any of the preceding claims.

15. An environmental sensor comprising a sensor and a processor adapted to execute the steps of the method according to any of claims 1-13,
in particular wherein the sensor comprises a MOX sensor, and/or
in particular wherein the sensor comprises a VOC sensor.

Amended claims in accordance with Rule 137(2) EPC.

1. Method for processing environmental sensor data, comprising the steps of

- receiving from an environmental sensor one or more raw data values (11),

- determining an average value and a measure of dispersion over a defined time period only from raw data values between a lower threshold and an upper threshold (13),
- redefining the lower threshold and the upper threshold (13) depending on the average value and the measure of dispersion,
- in response to a gating adaptation event (36) determining the average value and the measure of dispersion dependent on the raw data value received for the corresponding time step even if not between the lower threshold and the upper threshold.

2. Method according to claim 1,
   wherein one of the raw data values is received per discrete time step, wherein the average value and the measure of dispersion are determined anew per time step dependent on the raw data value received at the corresponding time step, wherein the lower threshold and the upper threshold (13) are redefined anew per time step dependent on the average value and the measure of dispersion determined for the corresponding time step,
   in particular wherein the lower threshold is the average value minus the measure of dispersion,
   in particular wherein the upper threshold (13) is the average value plus the measure of dispersion,
   in particular wherein the measure of dispersion corresponds to one or two times a standard deviation of the raw data values, and/or
   in particular wherein the average value corresponds to an arithmetic mean of the raw data values, and/or
   in particular wherein the defined time period is at least one hour, preferably at least one day, preferably between one and three days.

3. Method according to any of the preceding claims, additionally comprising the steps of

   - determining normalized data values from the raw data values depending on the average value and/or the measure of dispersion, and
   - outputting the normalized data values,
   - preferably determining the normalized data value anew per time step from the corresponding raw data value depending on the average value and/or the measure of dispersion determined for the corresponding time step.

4. Method according to any of the preceding claims,
   wherein the average value and the measure of dispersion are determined recursively each,
   in particular wherein the average value and the measure of dispersion are determined by exponential smoothing functions each,
   in particular wherein the average value is determined by $avt = \alpha * av_{t-1} + (1-\alpha) * rdv_t$
   with avt as average value at time t, $av_{t-1}$ as average value at time t-1, $\alpha$ as smoothing factor, and $rdv_t$ as raw data value received at time t,
   in particular wherein the measure of dispersion is determined by

   $$\sigma_t = \sqrt{\alpha * (\sigma^2_{t-1} + (1-\alpha)(rdv_t - av_{t-1})^2)}$$ with $\sigma_t$ as standard deviation at discrete time step t,

   $\sigma_{t-1}$ as standard deviation at discrete time step t-1, $av_{t-1}$ as average value at discrete time step t-1, $\alpha$ as smoothing factor, and $rdv_t$ as raw data value received at discrete time step t.

5. Method according to any of the preceding claims,
   wherein in response to a gating adaptation event (36), between the gating adaptation event and a gating adaptation disengagement event (38) and as long as the defined time period exceeds an interval between the gating adaptation event (36) and the gating adaptation disengagement event (38), the average value and the measure of dispersion are determined dependent on all raw data values even if not between the lower threshold and the upper threshold received on or after the gating adaptation event (36), and preferably dependent on the last average value and the last measure of dispersion determined prior to the gating adaptation event (36).

6. Method according to claim 5,

   - determining for a monitoring time period (37) a ratio between a number of raw data values that are not between the lower threshold and the upper threshold and a total number of raw data values, and
   - setting the gating adaptation event (36) if the ratio is larger than a maximum ratio.

**7.** Method according to claim 5,

- setting the gating adaptation event (36) if for a monitoring time period (37), no raw data value is between the lower threshold and the upper threshold.

**8.** Method according to any of the preceding claims 5 to 7,

- setting the gating adaptation disengagement event (38) after a predefined period in time (39) since the gating adaptation event (36),
- in response to the gating adaptation disengagement event (38)

determining the average value and the measure of dispersion dependent on the raw data value received for the corresponding time step only if between the lower threshold and the upper threshold,
in particular, after the gating adaptation disengagement event (38), determining the average value and the measure of dispersion per time step dependent only from the raw data values between the lower threshold and the upper threshold and received after the gating adaptation disengagement event (38), and preferably dependent on the last average value and the last measure of dispersion determined prior to the gating adaptation disengagement event (38).

**9.** Method according to any of the preceding claims, additionally comprising the steps of

- determining weights of a weighting function for the raw data values depending on the average value and the measure of dispersion,
- applying the weights to the raw data values when determining the average value and the measure of dispersion.

**10.** Method according to claim 9,
wherein the weights are largest at or around the average value, and
in particular wherein the weights increase monotonically for raw data values between zero and the average value, and/or
in particular wherein the weights decrease monotonically for raw data values between the average value and infinity.

**11.** Method for processing environmental sensor data according to any of the preceding claims, comprising the steps of

- at the beginning, receiving initial values for the lower threshold and the upper threshold,
- iterating the steps of the method according to any of the preceding claims.

**12.** Method for processing environmental sensor data according to any of the preceding claims, comprising the steps of

- at the beginning, receiving from the environmental sensor initial raw data values,
- determining an average value and a measure of dispersion from the initial raw data values,
- defining a lower threshold and an upper threshold depending on the average value and the measure of dispersion,
- iterating the steps of the method according to any of claims 1 to 10.

**13.** Method for processing environmental sensor data according to any of the preceding claims, comprising the steps of
in response to the receiving of the raw data value, determining if the received raw data value is between the lower threshold and the upper threshold,
in response to determining if the received raw data value is between the lower threshold and the upper threshold determining the average value and the measure of dispersion over the defined time period only from raw data values between the lower threshold and the upper threshold thereby including the received raw data value only if between the lower threshold and the upper threshold, thereby excluding the received raw data value from the determination of the average value and the measure of dispersion if not between the lower threshold and the upper threshold,
in response to determining the average value and the measure of dispersion redefining the lower threshold and the upper threshold depending on the determined average value and the determined measure of dispersion.

**14.** A computer program comprising instructions which, when the program is executed by a processor, cause the processor to execute the steps of the method according to any of the preceding claims.

**15.** An environmental sensor comprising a sensor and a processor adapted to execute the steps of the method according to any of claims 1-13,
in particular wherein the sensor comprises a MOX sensor, and/or
in particular wherein the sensor comprises a VOC sensor.

Fig. 1

Fig. 2

value

changed
environment
35

raw signal 31

38

34
threshold (robust gating)

36

...then adapt state estimation

33
threshold (gating)

37  39

wait max. gating duration...

time

## Fig. 3

S1

C2 — yes

no

C1 — yes

no

no — C3

yes

S6

S7 — yes — C4

no

S2

S3

## Fig. 4

## Fig. 5a

value

52a

53
threshold (gating)

52b
decay if signal
below threshold

52a
output signals

x raw signals   51a,b

output signals
52b

time

52a,b

54
threshold (robust gating)

no/similar decay
with smooth threshold

x raw signals   51a,b

output signals

52a,b

time

## Fig. 5b

weight

1

0

lt        av        ut

x

## Fig. 6

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/266088 A1 (OYAMADA HIDEO [JP]) 30 October 2008 (2008-10-30) * the whole document * | 1-4,9-15 | INV. G01N33/00 G08B29/26 |
| X | US 5 909 178 A (BALCH BRENT F [US] ET AL) 1 June 1999 (1999-06-01) * the whole document * | 1-4,9-15 | |
| X | KR 2019 0105218 A (NEXCERIS INNOVATION HOLDINGS LLC [US]) 16 September 2019 (2019-09-16) * the whole document * | 1-4,9-15 | |
| X | US 2003/065409 A1 (RAETH PETER G [US] ET AL) 3 April 2003 (2003-04-03) * the whole document * | 1-4,9-15 | |

TECHNICAL FIELDS
SEARCHED    (IPC)

G01N
G08B

The present search report has been drawn up for all claims

| Place of search | Date of completion of the search | Examiner |
|---|---|---|
| Munich | 14 May 2020 | Klein, Marc-Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 19 21 3789

This annex lists the patent family members relating to the patent documents cited in the above-mentioned European search report.
The members are as contained in the European Patent Office EDP file on
The European Patent Office is in no way liable for these particulars which are merely given for the purpose of information.

14-05-2020

| Patent document cited in search report | | Publication date | Patent family member(s) | | Publication date |
|---|---|---|---|---|---|
| US 2008266088 | A1 | 30-10-2008 | JP | 4353989 B2 | 28-10-2009 |
| | | | JP | 2008276473 A | 13-11-2008 |
| | | | US | 2008266088 A1 | 30-10-2008 |
| US 5909178 | A | 01-06-1999 | NONE | | |
| KR 20190105218 | A | 16-09-2019 | CN | 110418962 A | 05-11-2019 |
| | | | JP | 2019530037 A | 17-10-2019 |
| | | | KR | 20190105218 A | 16-09-2019 |
| US 2003065409 | A1 | 03-04-2003 | NONE | | |

For more details about this annex : see Official Journal of the European Patent Office, No. 12/82